# EUROPEAN PATENT APPLICATION

(11) **EP 2 819 044 A1**
(43) Date of publication of application: **31.12.2014**
(21) Application number: 14173650.4
(22) Date of filing: 24.06.2014
(51) Int. Cl.: G06F 19/00

(54) **Method and apparatus for providing user interface for medical imaging**

(30) Priority: 25.06.2013 KR 20130073313
(71) Applicant: Samsung Electronics Co., Ltd, Gyeonggi-do 443-742 (KR)
(72) Inventor: Oh, Keum-yong, 5204-1703 Gyeonggi-do (KR); Kang, Jeong-a, 945-404 Gyeonggi-do (KR); Kim, Dae-hwan, 5405-2104 Gyeonggi-do (KR); Kim, Hong-bum, 7-65 Seoul (KR); Min, Seung-ki, 854-102 Gyeonggi-do (KR); Bae, Jin-hyun, 336-1201 Gyeonggi-do (KR); Sung, Ki-yup, 108-1201 Gyeonggi-do (KR); Song, Mi-sook, 134-1601 Gyeonggi-do (KR); Yeo, Jae-gon, 9 Gwangju (KR); Lee, Min-ha, 13-513 Incheon (KR); Jo, Jae-moon, C-2803 Gyeonggi-do (KR)
(74) Representative: Lubberdink, Pim

(57) **Abstract**

A method for providing a UI for medical imaging of an object includes: generating UIs respectively corresponding to processes for imaging the object; determining a display order of the UIs; receiving a UI completion input of the medical professional regarding the first UI; and displaying the second UI in response to the receiving the UI completion input. The UIs corresponding to the processes for imaging the object may be automatically provided to the medical professional, based on the order.

## Description

### CROSS-REFERENCE TO RELATED APPLICATION

This application claims priority from Korean Patent Application No. 10-2013-0073313, filed on June 25, 2013, in the Korean Intellectual Property Office, the disclosure of which is incorporated herein in its entirety by reference.

### BACKGROUND

### 1. Field

One or more exemplary embodiments relate to providing a user interface (UI) for medical imaging of an object, and more particularly, to automatically providing a user interface for capturing a medical image.

### 2. Description of the Related Art

Since imaging of an object by using a medical imaging device may affect the health of the object, the method has to be performed very carefully. In particular, when the object is imaged by using electromagnetic waves in magnetic resonance imaging (MRI) or the like, it is important to adjust the amount of electromagnetic waves according to physical characteristics of the object.

In order to perform a plurality of processes for imaging an object, a medical professional has to directly execute each of programs. Since the medical professional directly executes each of the programs of the processes, it takes a long time to end and execute each of the programs and the medical professional has to know a method and an order of executing the programs.

### SUMMARY

Exemplary embodiments may address at least the above problems and/or disadvantages and other disadvantages not described above. The exemplary embodiments are not required to overcome the disadvantages described above, and may not overcome any of the problems described above.

One or more exemplary embodiments relate to a method, apparatus, and recording medium for automatically providing processes for capturing a medical image of an object to a medical professional by using a medical imaging device.

### BRIEF DESCRIPTION OF THE DRAWINGS

The above and/or other aspects will become more apparent by describing certain exemplary embodiments with reference to the accompanying drawings, in which:
FIG. 1 is a schematic view illustrating a system for providing to a medical professional a user interface for imaging an object by using a medical imaging device, according to an exemplary embodiment;
FIG. 2 is a flowchart of a method performed by a device to provide to the medical professional user interfaces for imaging the object by using the medical imaging device, according to an exemplary embodiment;
FIG. 3 is a detailed flowchart of a method performed by the device of FIG. 2 to determine a display order of a plurality of user interfaces;
FIG. 4 is a detailed flowchart of a method performed by the device of FIG. 2 to display a user interface when an input of the medical professional is completed;
FIG. 5 is a detailed flowchart of a method performed by the device of FIG. 2 to display a user interface according to an input of the medical professional;
FIG. 6 is a flowchart of a method performed by the device to provide to the medical professional a user interface for imaging the object by using the medical imaging device, according to another exemplary embodiment;
FIG. 7 is a view illustrating a user interface of a process of registering information of the object, according to an exemplary embodiment;
FIG. 8 is a view illustrating a user interface of a process of planning imaging, according to an exemplary embodiment;
FIG. 9 is a view illustrating a user interface of a process of controlling imaging, according to an exemplary embodiment;
FIG. 10 is a view illustrating a user interface of a process of displaying a result of imaging, according to an exemplary embodiment;
FIGS. 11A, 11B, and 11C are flowcharts for explaining imaging modes according to an exemplary embodiment;
FIG. 12 is a view illustrating a plurality of user interfaces based on an imaging method in an advanced mode, according to an exemplary embodiment;
FIGS. 13A and 13B are views for explaining a method performed by the device to provide to the medical professional a user interface for imaging the object, according to an exemplary embodiment;
FIG. 14 is a view illustrating a user interface in an automatic mode, according to an exemplary embodiment; and
FIG. 15 is a block diagram illustrating the device according to an exemplary embodiment.

### DETAILED DESCRIPTION

Certain exemplary embodiments are described in greater detail below with reference to the accompanying drawings.

In the following description, like drawing reference numerals are used for the like elements, even in different drawings. The matters defined in the description, such as detailed construction and elements, are provided to assist in a comprehensive understanding of exemplary embodiments. However, exemplary embodiments can be practiced without those specifically defined matters. Also, well-known functions or constructions are not described in detail since they would obscure the application with unnecessary detail.

Throughout the specification, when an element is referred to as being "connected" to another element, it may be "directly connected" to the other element or "electrically connected" to the other element with intervening elements therebetween. When a part "includes" or "comprises" an element, unless otherwise defined, the part may further include other elements, not excluding the other elements. As used herein, the term "and/or" includes any and all combinations of one or more of the associated listed items. Expressions such as "at least one of," when preceding a list of elements, modify the entire list of elements and do not modify the individual elements of the list.

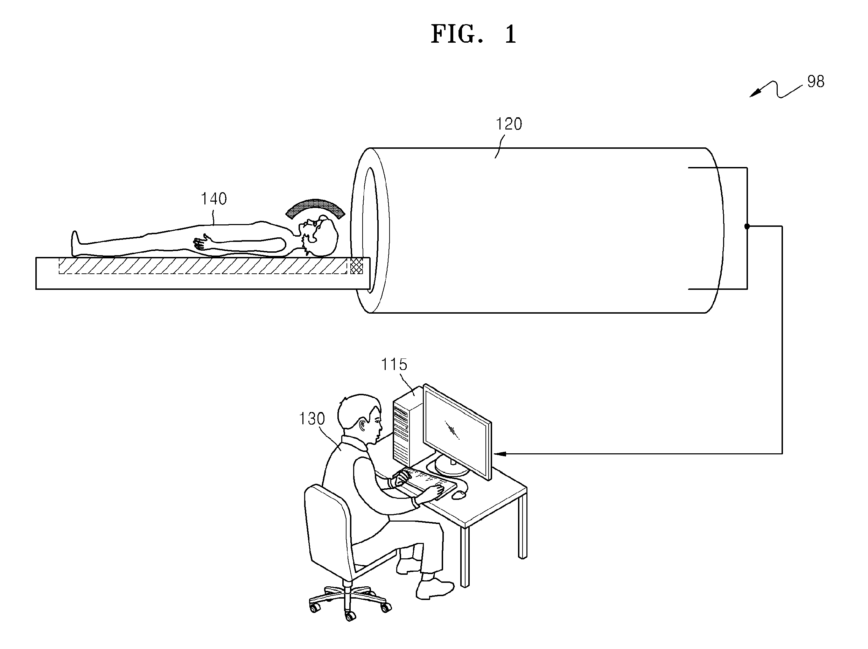

FIG. 1 is a schematic view illustrating a system 98 for providing to a medical professional 130 a user interface for imaging an object 140 by using a medical imaging device 120, according to an exemplary embodiment.

Referring to FIG. 1, the system may include a device 115 and the medical imaging device 120. The medical professional 130 may input pieces of information needed to image the object 140 through a user interface provided by the device 115.

The medical professional 130 has to perform a plurality of processes for inputting the pieces of information needed to image the object 140 in order to capture a medical image of the object 140. In FIG. 1, the device 115 may provide to the medical professional 130 a plurality of user interfaces respectively corresponding to the plurality of processes to be performed by the medical professional 130.

The medical professional 130 may input the pieces of information needed to image the object 140 through the plurality of user interfaces provided by the device 115. The plurality of processes may include, for example, a process of registering information of the object 140, a process of planning imaging, a process of controlling the imaging, a process of displaying a result of the imaging, and a process of outputting the result of the imaging.

Items which the medical professional 130 is to input in order to image the object 140 in the processes corresponding to the user interfaces provided by the device may be displayed on the user interfaces. Also, information about types of the plurality of processes for imaging the object 140 and an execution order may be displayed on the user interfaces.

The device 115 may transmit to the medical imaging device 120 the pieces of information input by the medical professional 130 through the user interfaces. The pieces of information may be transmitted through a plurality of communication interfaces for wireless communication and other types of communication interfaces for wired communication. For example, a plurality of communication interfaces for wireless communication such as infrared communication interfaces and Bluetooth wireless communication interfaces may be provided.

The medical imaging device 120 may image the object 140 based on the pieces of information received from the device 115.

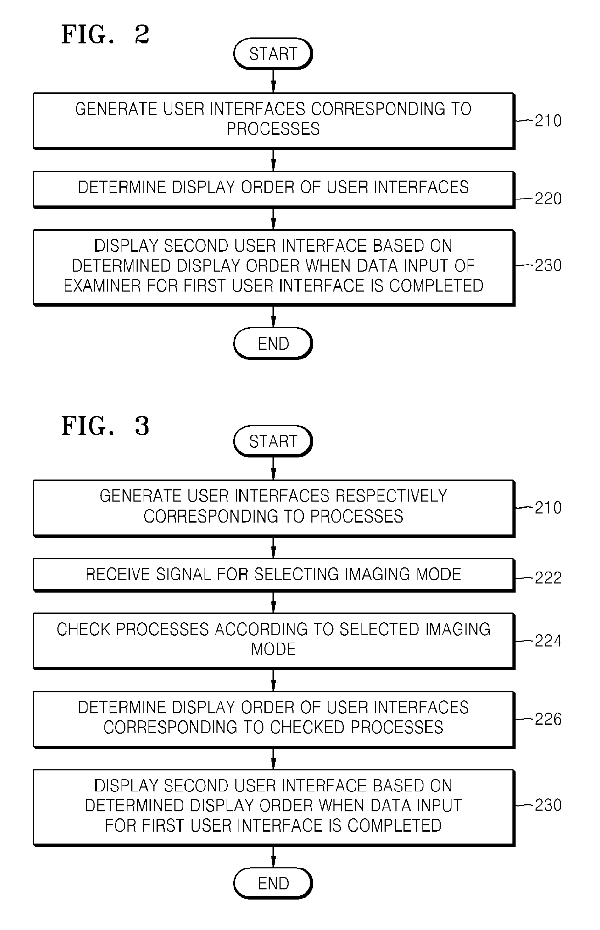

FIG. 2 is a flowchart of a method performed by the device 115 to provide to the medical professional 130 user interfaces for imaging the object 140 by using the medical imaging device 120, according to an exemplary embodiment.

In operation 210, the device 115 generates a plurality of user interfaces respectively corresponding to a plurality of processes for imaging the object 140. In FIG. 2, the plurality of processes may include a process of registering information of the object 140, a process of planning imaging, a process of controlling the imaging, and a process of displaying a result of the imaging. The processes are not limited thereto, and for example, the processes may further include a process of outputting the result of the imaging to another device.

The process of registering the information of the object 140 may be a process performed by the medical professional 130 to input personal information of the object 140 through a user interface. In detail, in the process of registering the information of the object 140, the device 115 may provide a user interface that may input personal information of the object 140 to the medical professional 130. The personal information of the object 140 may include information, for example, a height, a weight, an age, and a name, of the object 140. The personal information may include information about, for example, a health issue and a body part to be diagnosed of the object 140.

The process of planning the imaging may be a process of setting pieces of information needed to image the object 140 in consideration of a body part of the object 140 and physical characteristics of the object 140 to be imaged by the medical professional 130 before a medical image is captured. The pieces of information needed to image the object 140 may include, for example, slice information, coil information, and information about a type of a protocol.

The process of controlling the imaging may be a process of controlling the medical imaging device 120 while the object 140 is being imaged. In the process of controlling the imaging, a medical image of the object 140 captured by using the medical imaging device 120 may be displayed on a user interface. Also, a list for determining a process of post-processing the captured medical image and a time for which the object 140 is imaged may be displayed.

In the process of displaying the result of the imaging, the medical image of the object 140 captured based on the pieces of information set by the medical professional 130 may be displayed.

In the process of outputting the result of the imaging, the medical image of the object 140 may be transmitted to a doctor who diagnoses the object 140.

In FIG. 2, information about types of the plurality of processes to be performed by the medical professional 130 and an execution order may be displayed on the user interfaces. The types of the plurality of processes and the execution order may be determined according to a mode selected by the medical professional 130 to image the object 140. An imaging mode according to an exemplary embodiment will be explained below with reference to FIG. 3.

Items of pieces of information which the medical professional 130 is to currently input may be displayed on the user interfaces. The medical professional 130 may be guided to receive the pieces of information needed for the processes according to the items that are to be currently input and are displayed on the user interfaces. Also, information about where a process which the medical professional 130 is currently performing from among the plurality of processes places in the execution order of the selected imaging mode may be displayed.

In operation 220, the device 115 may determine a display order of the plurality of user interfaces. In FIG. 2, the plurality of user interfaces may be sequentially displayed in an order that is preset. For example, when the medical professional 130 selects an advanced mode, a process of registering information of the object 140, a process of planning imaging, a process of controlling the imaging, and a process of displaying a result of the imaging may be sequentially displayed in an order that is preset in the advanced mode. An execution order of the plurality of user interfaces is not limited thereto, and the plurality of user interfaces may be displayed in an order that is arbitrarily set by the medical professional 130.

In operation 230, the device 115 may display a second user interface based on the order determined in operation 220 as an input of the medical professional 130 for a first user interface is completed. The first user interface may be any one from among the plurality of user interfaces respectively corresponding to the plurality of processes. In detail, it may be assumed that a current process is a process of registering information of the object 140. The device 115 may provide a plurality of user interfaces that may input personal information such as a name, an age, or body information of the object 140 in the process of registering the information of the object 140. It may be assumed that the plurality of user interfaces are sequentially displayed such that information is input in an order of a name, an age, and body information. For example, assuming that a user interface that inputs an age of the object 140 is the first user interface that is currently being displayed, when the medical professional 130 completes an input of a name, the medical professional 130 may display a user interface that inputs an age, as the second user interface.

When the first user interface is a user interface that is displayed last from among the plurality of user interfaces corresponding to the process of registering the information of the object 140, a user interface corresponding to a next process may become the second user interface. In detail, in the advanced mode, a user interface that is displayed first from among the plurality of user interfaces corresponding to the process of planning the imaging may be displayed as the second user interface.

When information about items that are preset from among the items displayed on the first user interfaces is completely input, the device 115 may determine that the input of the medical professional 130 for the first user interface is completed. Also, when an input completion mark displayed on the first user interface is selected by the medical professional 130, the device 115 may determine that the input of the medical professional 130 for the first user interface is completed.

FIG. 3 is a detailed flowchart of a method performed by the device 115 of FIG. 2 to determine a display order of a plurality of user interfaces.

In operation 210, the device 115 generates a plurality of user interfaces respectively corresponding to a plurality of processes for imaging the object 140, as described above.

In operation 222, the device 115 may receive a mode selection signal for selecting an imaging mode from the medical professional 130. The imaging mode may be designated according to types of the processes needed in order for the medical professional 130 to image the object 140.

The medical professional 130 may receive a list of a plurality of imaging modes. The list of the plurality of imaging modes may be displayed on the user interfaces in at least one of a text, an image, and graphics. In FIG. 3, the medical professional 130 may select an imaging mode suitable for an objective to image the object 140 from among the plurality of imaging modes displayed on the user interfaces.

In operation 224, the device 115 may check a plurality of processes according to the imaging mode selected by the medical professional 130. In FIG. 3, the imaging mode may include a basic mode, an advanced mode, an analysis mode, and an automatic mode.

The basic mode may include a process of registering information of the object 140, a process of controlling imaging, a process of displaying a result of the imaging, and a process of outputting the result of the imaging. Since imaging is automatically planned by using registered data of the object 140 in the basic mode, a process of planning imaging may be omitted.

In detail, in the basic mode, information about an imaging plan which is preset may be loaded based on personal information of the object 140 which is preset. The preset information about the imaging plan may be stored in the device 115. Alternatively, a separate server that stores the preset information about the imaging plan may exist. When the separate server exists, the device 115 may obtain setting information of the medical imaging device 120 which is preset by communicating with the separate server.

The advanced mode may include a process of registering information of the object 140, a process of planning imaging, a process of controlling the imaging, a process of displaying a result of the imaging, and a process of outputting the result of the imaging.

The advanced mode may be used when the medical professional 130 needs to directly set the information about the imaging plan of the object 140. For example, when a physical structure of the object 140 is different from a general structure or when imaging is performed based on physical characteristics of the object 140, the medical professional 130 may plan direct imaging. As another example, when a body part of the object 140 has to be repeatedly imaged or when various experiments have to be performed in order to find a cause of a health problem, the medical professional 130 may plan direct imaging.

The analysis mode may include a process of registering information of the object 140, a process of planning imaging, a process of controlling the imaging, a process of displaying a result of the imaging, a process of analyzing the result of the imaging, and a process of outputting the result of the imaging.

The analysis mode may be used when clinical analysis of the result of the imaging is performed. For example, when an additional medical image, such as a functional magnetic resonance imaging (fMRI) image, a diffusion tenor imaging (DTI) image, or a perfusion-weighted image needs to be analyzed, a captured image may be analyzed by selecting the analysis mode.

When the automatic mode is selected, the device 115 may recommend a list displayed on the user interfaces such that only a function often used to capture a medical image is displayed on the user interfaces. In detail, in the automatic mode, a list of information that is input a number of times equal to or greater than a number of times that is preset by the medical professional 130 in the processes may be recommended and displayed on the user interfaces. For example, information that is used N times may be displayed, and information that is used a number of times less than N times may be searched through a search window on the user interfaces.

In operation 226, the device 115 determines a display order of user interfaces corresponding to the processes checked in operation 224. For example, when the medical professional 130 selects the basic mode, the device 115 determines a display order of user interfaces corresponding to a process of registering information of the object 140, a process of controlling imaging, a process of displaying a result of the imaging, and a process of outputting the result of the imaging. The device 115 may check a display order that is preset for the basic mode, and may determine a display order of the user interfaces based on the checked display order.

In operation 230, the device 115 may display a second user interface based on the determined display order as a user input regarding a first user interface is completed. Examples of the second user interface may include a user interface that is displayed after the first user interface according to the determined display order.

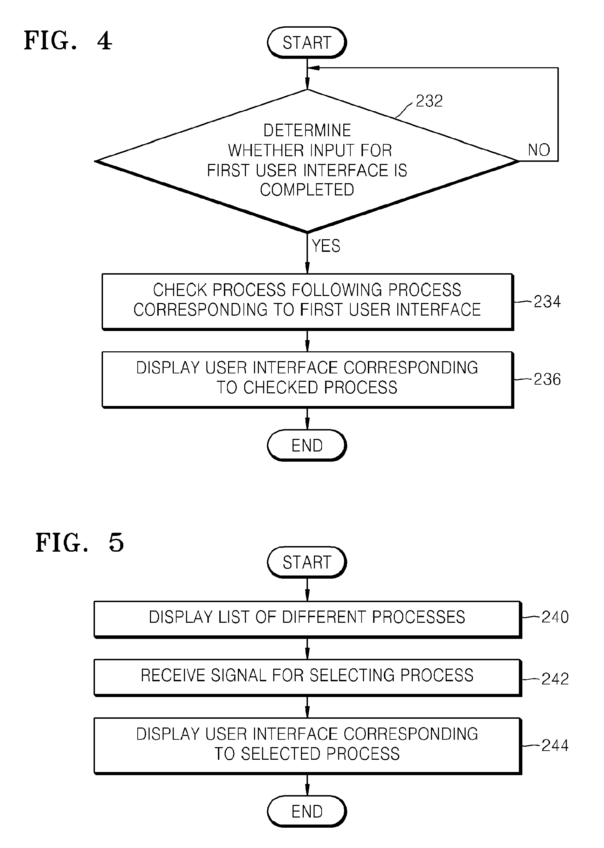

FIG. 4 is a detailed flowchart of a method performed by the device 115 to display a second user interface based on a preset order, when the medical professional 130 completes an input for a current process.

In operation 232, the device 115 determines whether an input of the medical professional 130 regarding a first user interface is completed.

In FIG. 4, when an input completion signal is received from the medical professional 130, the device 115 may determine that the input of the medical professional 130 regarding the first user interface is completed. The medical professional 130 may generate the input completion signal by selecting an input completion mark provided on the first user interface. The input completion mark may be displayed in at least one of a text, an image, and a video.

The medical professional 130 may select the input completion mark by using an input device such as a mouse or a keyboard. Alternatively, the medical professional 130 may select the input completion mark by touching the input completion mark of the first user interface.

In operation 234, when the input completion signal is received from the medical professional 130, the device 115 checks a second user interface that is displayed after the first user interface.

In operation 236, the device 115 displays the second user interface that corresponds to the checked process.

FIG. 5 is a detailed flowchart of a method performed by the device 115 of FIG. 2 to display a user interface according to an input of the medical professional 130.

In operation 240, the device 115 displays a list of a plurality of processes for imaging the object 140. Types of processes and an execution order according to an imaging mode selected by the medical professional 130 may be displayed in the list of the plurality of processes. In FIG. 5, the list of the plurality of processes may be displayed in at least one of a text, an image, and a video. The device 115 may display the current process distinguishably from other processes so that the medical professional 130 knows a position of the current process from among the plurality of processes.

In operation 242, the device 115 receives a signal for selecting one process from among the plurality of processes from the medical professional 130. The signal for processing one process may be different from the input completion signal in operation 232 of FIG. 4.

The signal for selecting one process may be generated even when an input of the medical professional 130 regarding a user interface is not completed. For example, while the medical professional 130 is setting an imaging plan of the object 140, when the medical professional 130 desires to check physical information of the object 140, the medical professional 130 may select a mark for a process of registering information of the object 140 displayed on a user interface.

The medical professional 130 may select a predetermined process by using an input device such as a mouse or a keyboard. The medical professional 130 may select a mark for the predetermined process.

In operation 244, the device 115 displays a user interface corresponding to the process selected in operation 242. For example, when the medical professional 130 selects a mark for registering information of the object 140, the device 115 may display a user interface corresponding to the process of registering the information of the object 140.

The medical professional 130 may end the current process by selecting a desired process from among the processes displayed on the user interface, and may receive a user interface corresponding to the desired process without an additional process of starting a new process.

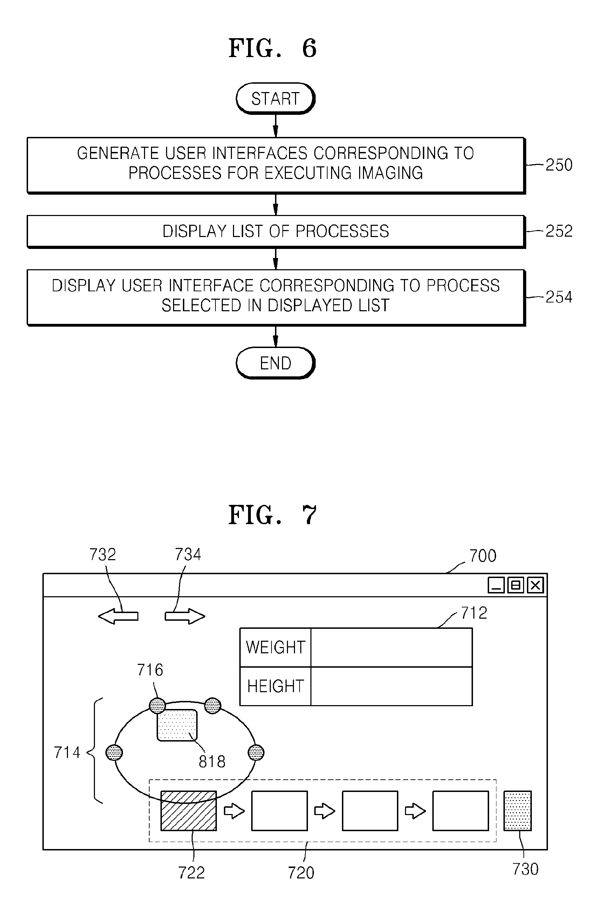

FIG. 6 is a flowchart of a method performed by the device 115 to provide to the medical professional 130 a user interface for imaging the object 140 by using the medical imaging device 120, according to another exemplary embodiment.

In operation 250, the device 115 generates a plurality of user interfaces respectively corresponding to a plurality of processes for imaging the object 140, as described above with reference to FIG. 2.

In operation 252, the device 115 displays a list of the plurality of processes. Types of the processes and an execution order may be displayed in the list of the plurality of processes. The list of the plurality of processes may be displayed in at least one of a text, an image, and a video.

The device 115 may display a description of each of the plurality of processes in order for the medical professional 130 to easily understand each process. In FIG. 6, when the medical professional 130 accesses a mark for a predetermined process by using an input device, a description of the predetermined process may pop up and be displayed.

In operation 254, the device 115 may display a user interface corresponding to a process selected by the medical professional 130 from among the plurality of processes based on a selection input of the medical professional 130 for the plurality of processes. The medical professional 130 may determine a display order of a plurality user interfaces corresponding to a plurality of selected processes.

The medical professional 130 may efficiently capture an image of the object 140 by selecting a plurality of processes suitable for imaging and one or more user interfaces corresponding to each of the plurality of processes and determining a display order of the user interfaces corresponding to each of the plurality of the selected processes.

FIG. 7 is a view illustrating a user interface 700 of a process of registering information of the object 140, according to an exemplary embodiment.

Referring to FIG. 7, the device 115 may receive information of the object 140 from the medical professional 130 in the process of registering the information of the object 140. The medical professional 130 may input personal information of the object 140 through the user interface 700 corresponding to the process of registering the information of the object 140. The personal information of the object 140 may include a name, an age, a gender, body information, health-related information, etc.

In FIG. 7, a plurality of input items 714 which the medical professional 130 is to input and a list 720 of a plurality of processes may be displayed. For example, a plurality of input items 714 may be displayed as UI objects connected by a curved line on or proximate to a UI corresponding to the process 722 of registering the information of the object 140.

An item 716 corresponds to an informational item that is to be currently input by the medical professional 130. As the item 716 to be currently input is displayed on a user interface, the medical professional 130 may receive pieces of information that need to be input in order to capture a medical image of the object 140.

In FIG. 7, the input items 714 to be currently input may be sequentially displayed in a preset order, i.e., the user interfaces that may serve to input the current corresponding items may be sequentially displayed. As another example, the medical professional 130 may select a predetermined item of the plurality of input items 714, and may receive a user interface that may serve to input the predetermined item, from the device 115. For example, an information input window 712 that may serve to input information about the predetermined item 716, for example, body information of the object, and may be displayed on the user interface 700. The medical professional 130 may input body information of the object 140, into an information input window 712, by using an input device such as a mouse or a keyboard. Alternatively, the medical professional 130 may input the body information of the object 140 by using a touchpad.

Processes included in an imaging mode selected by the medical professional 130 may be displayed in the list 720 of the plurality of processes for imaging the object 140. For example, when the imaging mode selected by the medical professional 130 is a basic mode, a process of registering information of the object 140, a process of controlling imaging, a process of displaying a result of the imaging, and a process of outputting the result of the imaging may be displayed in the list 720 of the plurality of processes for imaging the object 140.

In FIG. 7, a process 722 corresponding to a user interface that is being displayed on the device 115 is displayed in the list 720 of the plurality of processes. The medical professional 130 may receive information about an order and types of the processes for imaging the object 140 and information about a position of a current process by using the list 720 of the plurality of processes.

When the medical professional 130 selects a mark, i.e., UI object, for a process to be displayed from among marks of processes displayed in the list 720 of the plurality of processes, a user interface corresponding to the selected process may be displayed. Also, when a mark for a predetermined item is selected from the plurality of input items 714, the device 115 may provide a user interface that may receive the predetermined item.

In FIG. 7, the medical professional 130 informs the device 115 that an input for the current process, e.g., process 722, is completed by selecting an input completion mark 730.

In FIG. 7, the device 115 may receive a movement signal from the medical professional 130. Examples of the movement signal may include a forward movement signal and a backward movement signal.

The medical professional 130 may transmit to the device 115 the forward movement signal by selecting a forward movement signal mark 734. The forward movement signal mark 734 may include a text, an image, or an icon.

When the received movement signal is a forward movement signal, a second user interface that is a user interface after a first user interface that is being currently displayed may be displayed based on the determined order. As an example, in a process of registering information of the object 140, the medical professional 130 inputs the information of the object 140 in an order of a name, an age, and body information. Assuming that the first user interface that is being currently displayed is a user interface that receives an age, when the device 115 receives a forward movement signal, a user interface that is a user interface after the first user interface and inputs body information may be displayed.

When a user interface that is being currently displayed is a user interface that places last in the order in a process of registering information of the object 140, a user interface that comes first in the order from among a plurality of user interfaces corresponding to a next process may be displayed. For example, in an advanced mode, a user interface that comes first in the order from among user interfaces corresponding to a process of planning imaging may be displayed.

The device 115 may receive a backward movement signal of the medical professional 130. The medical professional 130 may transmit to the device 115 a backward movement signal by selecting a backward movement signal mark 732. The backward movement signal mark 732 may include a text, an image, a video, or an icon.

When the received movement signal is a backward movement signal, a user interface before the first user interface that is being currently displayed may be displayed based on the determined order. In detail, it may be assumed that in a process of registering information of the object 140, the medical professional 130 inputs information of the object 140 in an order of a name, an age, and body information. Assuming that the first user interface that is being currently displayed is a user interface that receives an age input, when the device 115 receives a backward movement signal, a user interface that is a user interface before the first user interface and inputs a name may be displayed.

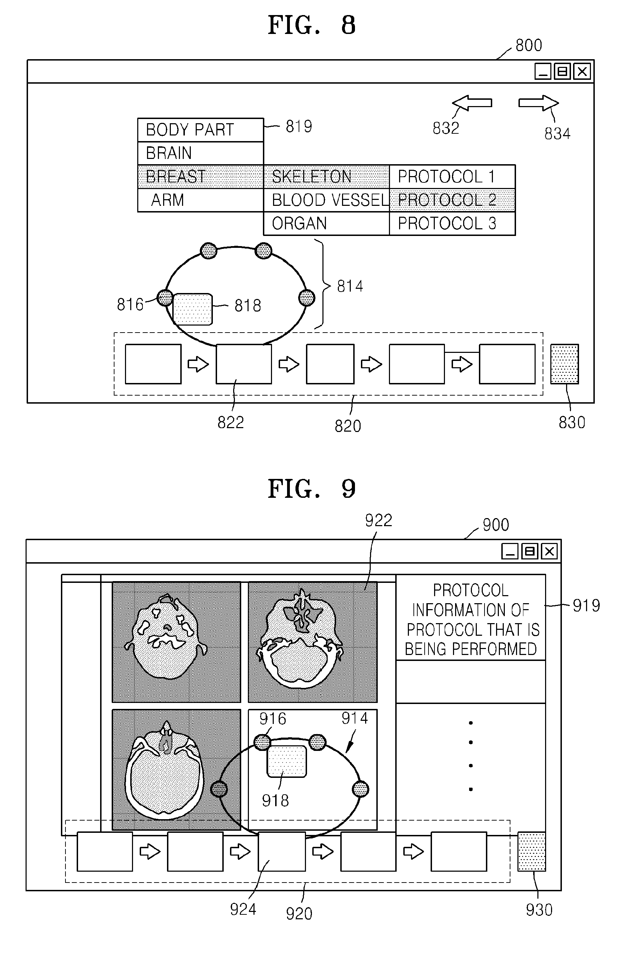

FIG. 8 is a view illustrating a user interface 800 of a process of planning imaging, according to an exemplary embodiment.

Referring to FIG. 8, the device 115 may receive information about an imaging plan of the object 140 from the medical professional 130 in the process of planning the imaging.

A plurality of input items 819 which need to be input to plan the imaging, and a list 820 about a plurality of processes may be displayed on user interfaces corresponding to the process 822 of planning the imaging.

An item 816 is an item which is to be currently input by the medical professional 130 and may be displayed in the plurality of input items 814. For example, items for setting coil information, selecting a slice, and setting a protocol may be displayed in plurality of input items 814 needed to plan imaging.

In FIG. 8, the items to be currently input may be sequentially displayed according to a preset order or an item 816 to be currently input may be arbitrarily selected by the medical professional 130, as described above.

In FIG. 8, when the medical professional 130 accesses one of the input items 814 to be currently input by using an input device, a description 818 of the selected item may be displayed on a popup window, for example, connectedly to the selected item or in close proximity to the selected item. For example, assuming that the item 816 to be currently input is an item for setting a protocol, when the medical professional 130 accesses the item 816 to be currently input by using an input device, the description of an input item for setting a protocol may be displayed.

In FIG. 8, the user interface 800 of the process of planning the imaging may divide a list of pieces of information needed to capture a medical image of the object 140 into a sub-list and a main list including the sub-list and may display the sub-list and the main list.

In detail, pieces of information about types of a plurality of protocols, a standard specification of a coil, and/or a slice, which are needed to plan imaging, may be displayed on the information input window 819. Also, since items needed to plan imaging vary according to a body part to be imaged and an imaging objective, a plurality of input items needed to plan imaging may be displayed on the information input window 819 according to a body part and an imaging objective.

For example, body parts of the object 140 to be imaged may be divided into body parts such as a brain, a breast, and an arm, the body parts may be displayed, and a sub-list of each of the body parts may be displayed. When a body part of the object 140 to be imaged is a breast, items such as a skeleton, a blood vessel, and an organ may be displayed as a sub-list of the breast. For example, when a skeleton is imaged, a protocol suitable for a breast skeleton may be set. In FIG. 8, a protocol may be separately displayed according to setting values of time repetition (TR), echo time (TE), and field of view (FOV).

Processes included in an imaging mode selected by the medical professional 130 may be displayed in the list 820 of the plurality of processes for imaging the object 140. For example, when the imaging mode selected by the medical professional 130 is an advanced mode, a process of registering information of the object 140, a process of planning imaging, a process of controlling the imaging, a process of displaying a result of the imaging, and a process of outputting the result of the imaging may be displayed in the list 820 of the plurality of processes for imaging the object 140.

In FIG. 8, the medical professional 130 may inform the device 115 that an input for a current process is completed by selecting an input completion mark 830.

In FIG. 8, the device 115 may receive a movement signal from the medical professional 130, as described above.

The medical professional 130 may transmit to the device 115 a forward movement signal by selecting a forward movement signal mark 834, as described above.

For example, it may be assumed that a user interface that may input information about a type of a protocol, a standard specification of a coil, and a slice may be sequentially displayed in a process of planning imaging.

Assuming that the first user interface that is being currently displayed is a user interface corresponding to a sub-process that inputs information about a standard specification of a coil, when the device 115 receives a forward movement signal, a user interface that is a user interface after the first user interface and receives information about a slice may be displayed.

The device 115 may receive a backward movement signal of the medical professional 130. The medical professional 130 may transmit to the device 115 a backward movement signal by selecting a backward movement signal mark 832, as described above.

For example, if the first user interface that is being currently displayed is a user interface that receives information about a standard specification of a coil, when the device 115 receives a backward movement signal, a user interface that is a user interface before the first user interface and inputs a type of a protocol may be displayed.

FIG. 9 is a view illustrating a user interface 900 of a process of controlling imaging, according to an exemplary embodiment.

Referring to FIG. 9, the device 115 may receive information about imaging control of the object 140 from the medical professional 130 in the process of controlling the imaging.

A plurality of input items 914, an information input window 919 which is to control imaging, a list 920 of a plurality of processes, and an image 922 that is being captured may be displayed on user interfaces corresponding to the process 924 of controlling the imaging.

An item 916 which the medical professional 130 is to currently input may be displayed in the plurality of input items 914 needed to control imaging. For example, items for protocol information of a protocol that is being currently performed, a progress state, and whether to start scanning may be displayed as the plurality of input items 914.

In FIG. 9, the items to be currently input may be sequentially displayed according to a preset order or may be selected by the medical professional 130, as described above. In FIG. 9, when the medical professional 130 accesses of the items 916 to be currently input by using an input device, a description 918 of the selected item 916 may be displayed on a popup window. For example, assuming that the item 916 to be currently input is an item for a protocol that is being currently performed, when the medical professional 130 accesses the item 916 to be currently input by using an input device, the description 918 of an input item, that is, the protocol that is being currently performed, may be displayed.

In FIG. 9, information about setting content of a protocol that is being currently performed and an execution order of imaging may be displayed on the information input window 919 needed to control imaging.

Processes included in an imaging mode selected by the medical professional 130 may be displayed in the list 920 of the plurality of processes for imaging the object 140. For example, when the imaging mode selected by the medical professional 130 is an advanced mode, a process of registering information of the object 140, a process of planning imaging, a process of controlling the imaging, a process of displaying a result of the imaging, and a process of outputting the result of the imaging may be displayed in the list 920 of the plurality of processes for imaging the object 140.

The image 922 that is being captured may be displayed on a user interface corresponding to the process of controlling the imaging.

The medical professional 130 may inform the device 115 that an input for a current process is completed by selecting an input completion mark 930.

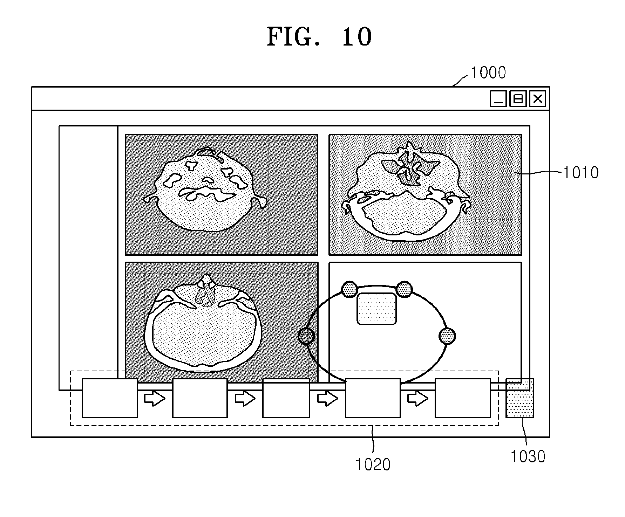

FIG. 10 is a view illustrating a user interface 1000 of a process of displaying a result of imaging, according to an exemplary embodiment.

Referring to FIG. 10, the device 115 may receive information about the result of the imaging from the medical imaging device 120 in the process of displaying the result of the imaging.

A result 1010 of imaging, obtained according to settings of the medical professional 130, may be displayed on a user interface corresponding to the process of displaying the result of the imaging.

Types of processes included in an imaging mode selected by the medical professional 130 and an execution order may be displayed in a list 1020 of a plurality of processes for imaging the object 140. For example, when the imaging mode selected by the medical professional 130 is an advanced mode, a process of registering information of the object 140, a process of planning imaging, a process of controlling the imaging, a process of displaying a result of the imaging, and a process of outputting the result of the imaging may be displayed in the list 1020 of the plurality of processes for imaging the object 140.

The medical professional 130 may inform the device 115 that an input for a current process is completed by selecting an input completion mark 1030.

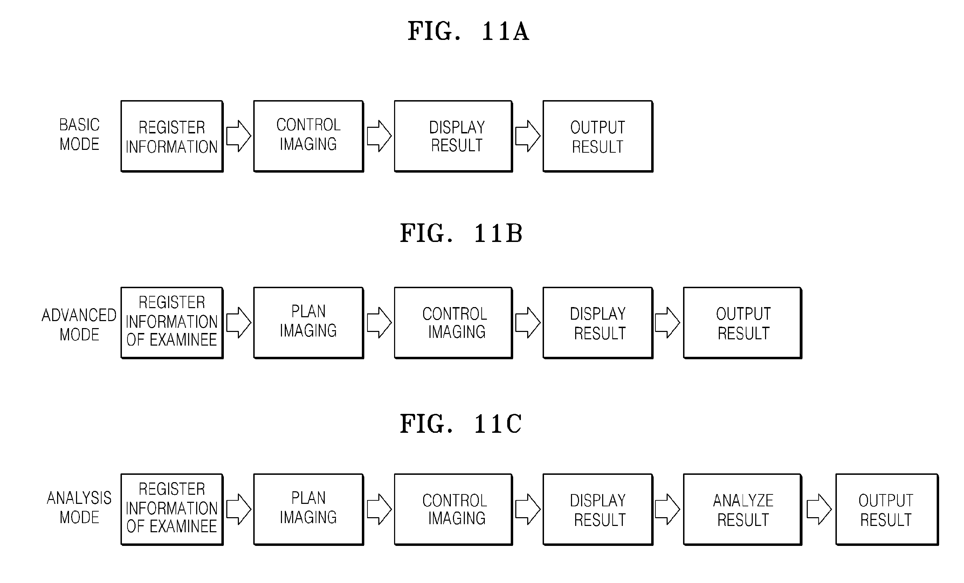

FIGS. 11A, 11B, and 11C are flowcharts for explaining imaging modes according to an exemplary embodiment. The imaging modes may include, for example, a basic mode, an advanced mode, an analysis mode, and an automatic mode.

In FIG. 11A, the basic mode may include a process of registering information of the object 140, a process of controlling imaging, a process of displaying a result of the imaging, and a process of outputting the result of the imaging. Since imaging is automatically planned by using data of the object 140 that is registered in the basic mode, a process of planning imaging may be omitted.

In FIG. 11B, the advanced mode may include a process of registering information of the object 140, a process of planning imaging, a process of controlling the imaging, a process of displaying a result of the imaging, and a process of outputting the result of the imaging.

The advanced mode may be used when the medical professional 130 needs to directly set information about an imaging plan, as described above.

In FIG. 11C, the analysis mode may include a process of registering information of the object 140, a process of planning imaging, a process of controlling the imaging, a process of displaying a result of the imaging, a process of analyzing the result of the imaging, and a process of outputting the result of the imaging.

The analysis mode may be used when clinical analysis of the result of the imaging is performed. For example, when an additional medical image such as an fMRI image, a DTI image, or a perfusion-weighted image needs to be analyzed, a captured image may be analyzed by selecting the analysis mode.

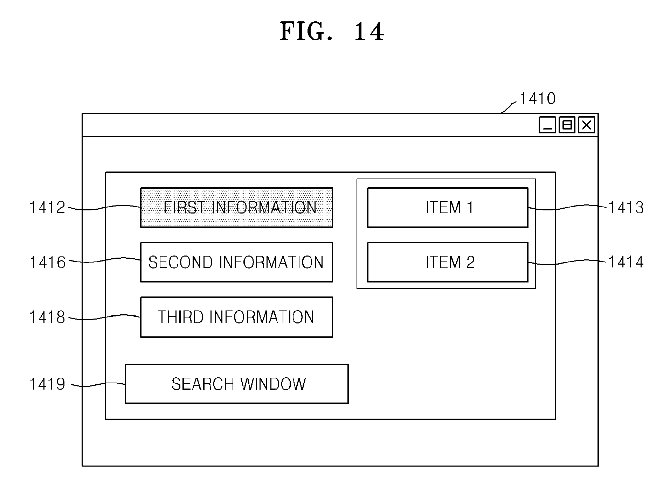

The automatic mode may recommend a list such that only a function often used to capture a medical image is displayed. The automatic mode will be explained below in detail with reference to FIG. 14.

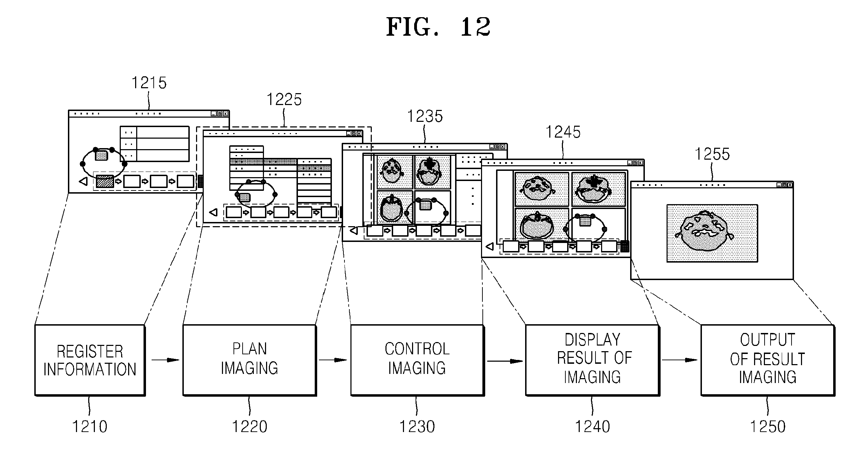

FIG. 12 is a view illustrating a plurality of user interfaces based on an imaging method in an advanced mode, according to an exemplary embodiment.

In FIG. 12, the device 115 may provide to the medical professional 130 a user interface 1215 corresponding to a process 1210 of registering information of the object 140, a user interface 1225 corresponding to a process 1220 of planning imaging, a user interface 1235 corresponding to a process 1230 of controlling the imaging, a user interface 1245 corresponding to a process 1240 of displaying a result of the imaging, and a user interface 1255 corresponding to a process 1250 of outputting the result of the imaging.

Since types of a plurality of processes and an execution order are displayed on each of the plurality of user interfaces, the medical professional 130 may check processes for capturing a medical image. The medical professional 130 may search for a user interface corresponding to another process in order to check information about the process other than a process that is being currently performed.

In detail, it may be assumed that a process that is being currently performed is the process 1220 of planning imaging. Referring to FIG. 8, the medical professional 130 may search for a plurality of user interfaces by selecting at least one item from the plurality of input items 814 needed to plan imaging, the list 820 of the plurality of processes, the forward movement signal mark 834, and the backward movement signal mark 832 displayed on the user interface 800 corresponding to the process 1220 of planning imaging. The user interface 800 of FIG. 8 may be the same as the user interface 1225 corresponding to the process 1220 of planning imaging of FIG. 12.

When the medical professional 130 selects the forward movement signal mark 834, a user interface after the user interface that is being currently displayed may be displayed. When the medical professional 130 selects the backward movement signal mark 832, a user interface before the user interface that is being currently displayed may be displayed.

The medical professional 130 may receive a user interface corresponding to a predetermined process by selecting a mark for the predetermined process to be moved in the list 820 of the plurality of processes. The medical professional 130 may receive a user interface that may input information about a predetermined item by selecting the predetermined item in the plurality of input items 814 needed to plan imaging in the process 1220 of planning imaging. For example, when the medical professional 130 selects an item that determines a protocol in the process 1220 of planning imaging, the medical professional 130 may receive a user interface that may input information about the protocol.

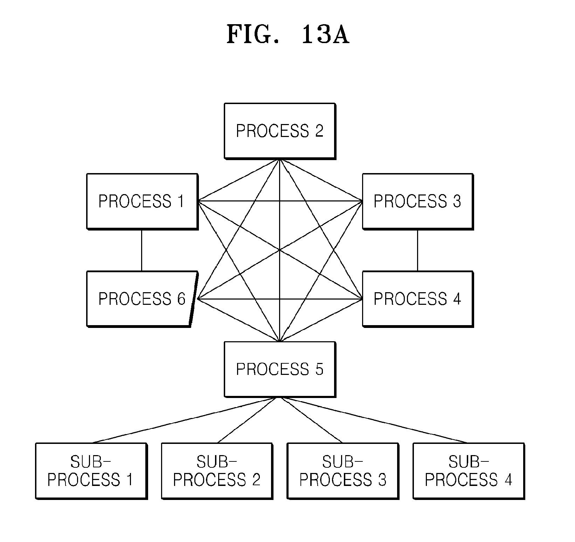

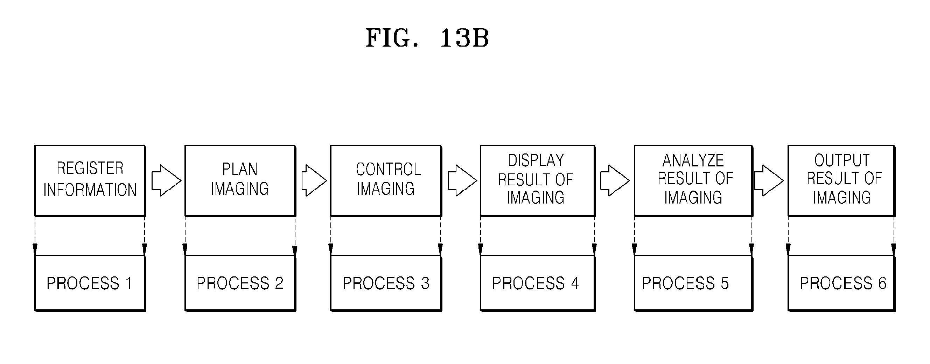

FIGS. 13A and 13B are views for explaining a method performed by the device 115 to provide to the medical professional 130 a user interface for imaging the object 140, according to an exemplary embodiment. The medical professional 130 may determine types of processes for capturing a medical image and an execution order in FIGS. 13A and 13B.

Referring to FIG. 13A, a process of registering information of the object 140 may correspond to a process 1, a process of planning imaging may correspond to a process 2, a process of controlling the imaging may correspond to a process 3, a process of displaying a result of the imaging may correspond to a process 4, a process of analyzing the result of the imaging may correspond to a process 5, and a process of outputting the result of the imaging may correspond to a process 6. FIG. 13A shows that all of the processes are interconnected and that the process 5 includes several sub-processes, but this is not limiting.

Referring to FIG. 13B, the medical professional 130 may select a plurality of processes for imaging the object 140. The medical professional 130 may determine an execution order of the selected processes.

For example, when the medical professional 130 images the object 140 who is already registered, the process 1 that is the process of registering information of the object 140 may be omitted. Also, when a medical image of the object 140 is re-captured for the same purpose, the process 1 that is the process of registering information of the object 140 and the process 2 that is the process of planning imaging may be omitted.

A plurality of processes may respectively correspond to a plurality of user interfaces. For example, the process 1 that is the process of registering information of the object 140 may correspond to a plurality of user interfaces that may respectively input a name, an age, and body information of the object 140. An age may be important in capturing a medical image of a child such as a newborn baby. However, when an age does not affect a process of capturing of a medical image, the medical professional 130 may select user interfaces that may input a name and body information.

FIG. 14 is a view illustrating a user interface 1410 in an automatic mode, according to an exemplary embodiment.

Referring to FIG. 14, lists 1412, 1416, and 1418 of information input a number of times equal to or greater than a preset number of times may be displayed on user interfaces. By contrast, functions used a number of times less than the preset number of times may be searched on the user interface through a search window 1419.

In detail, in a process of registering information of a patient, since, for example, a height and a weight determine the amount of electromagnetic waves applied to a body of the object 140, items that set the height and the weight are information that needs to be variously input according to the object 140. Hence, the items that set the height and the weight may be assumed to be functions used N times or more. By contrast, an age is input a less number of times than the height and the weight. Hence, an item that sets the age may be assumed to be a function used a number of times less than N times.

In FIG. 14, when an automatic mode is selected, in a process of registering information of the object 140, items related to a height and a weight which are functions used N times or more may be displayed, whereas an item related to an age which is a function used a number of times less than N times will not be displayed. When the medical professional 130 is to set the age, the medical professional 130 may search for the item related to the age through the search window 1419.

In FIG. 14, when the medical professional 130 accesses the item 1412 for first information by using an input device or a touch, items 1413 and 1414 for pieces of information related to the item 1412 for the first information may be displayed on a popup window.

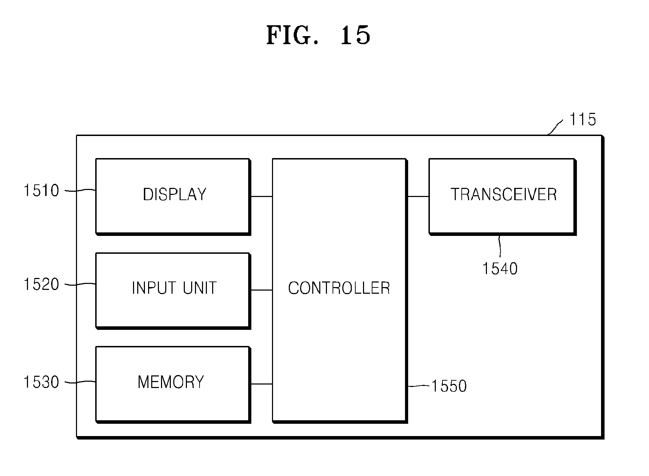

FIG. 15 is a block diagram illustrating the device 115 according to an exemplary embodiment.

Referring to FIG. 15, the device 115 includes a display 1510, an input unit 1520, a memory 1530, a transceiver 1540, and a controller 1550.

The display 1510 may display a plurality of user interfaces corresponding to a plurality of processes for imaging the object 140. Pieces of information needed to image the object 140 may be input from the medical professional 130 to the displayed user interfaces.

The input unit 1520 may receive the information input by the medical professional 130. The medical professional 130 may input the information to a user interface corresponding to a predetermined process by using an input device such as a mouse or a keyboard. Alternatively, the medical professional 130 may input the information to the user interface by touching the display 1510. In the latter case, the input unit 1520 may detect a touch input of the medical professional 130 by using a touch sensor.

The memory 1530 may store the information input by the medical professional 130. Also, in FIG. 15, the memory 1530 may store preset setting information of the medical imaging device 120, which is used in a basic mode.

The transceiver 1540 may transmit to the medical imaging device 120 the pieces of information input by the medical professional 130 in the plurality of processes for imaging the object 140, and may transmit to the device 115 an image that is being captured to provide information about an imaging state to the medical professional 130. Also, in a process of outputting a result of the imaging, the transceiver 1540 may transmit the result of the imaging that is received from the medical imaging device 120 to another server.

The controller 1550 may control an overall operation of the device 115, and may control the display 1510, the input unit 1520, the memory 1530, and the transceiver 1540 to provide the user interfaces corresponding to the plurality of processes for imaging the object 140.

The controller 1550 may generate the plurality of user interfaces respectively corresponding to the plurality of processes for imaging the object 140. The controller 1550 may determine a display order of the generated plurality of user interfaces.

When all of the inputs of the medical professional 130 regarding a first user interface from among the plurality of user interfaces are completed, the controller 1550 displays a second user interface from among the plurality of user interfaces based on the determined display order. The plurality of processes may include a process of registering information of the object 140, a process of planning imaging, a process of controlling the imaging, a process of displaying a result of the imaging, and a process of outputting the result of the imaging.

The controller 1550 may receive a signal for selecting an imaging mode of the medical imaging device 115 from the medical professional 130 in order to determine a display order of the plurality of user interfaces. The imaging modes are divided according to types of the processes for imaging the object 140. The imaging mode may include at least one of a basic mode, an advanced mode, an analysis mode, and an automatic mode. In the automatic mode, the device 115 may recommend to a user interface a list of information input a number of times equal to or greater than a number of times that is preset by the medical professional 130 in each process.

When the controller 1550 receives an input completion signal for a current process from the medical professional 130, the controller 1550 may display a user interface corresponding to a process after the current process based on the determined display order. When the controller 1550 receives a signal for selecting one of the plurality of processes, the controller 1550 may display a user interface corresponding to the selected process.

The medical professional 130 may input information needed to perform the process corresponding to the user interface through the user interface. The plurality of processes for imaging the object 140 and the determined display order may be displayed on the user interface.

In FIG. 15, the controller 1550 may generate a plurality of user interfaces respectively corresponding to a plurality of processes for imaging the object 140, and may display a list of the plurality of processes. When the medical professional 130 selects predetermined processes in the displayed list, the controller 1550 may display user interfaces corresponding to the predetermined processes selected by the medical professional 130 from among the plurality of processes based on a selection input of the medical professional 130.

The controller 1550 may provide a user interface for imaging the object 140 by using the medical imaging device 120 by executing at least one program stored in the memory 1530. The at least one program stored in the memory 1530 may include commands for executing each of the methods of FIGS. 2 through 6.

Exemplary embodiments may be embodied as a recording medium, e.g., a program module to be executed in computers, which include computer-readable commands. The computer storage medium may include any usable medium that may be accessed by computers, volatile and non-volatile media, and detachable and non-detachable media. The computer storage medium may include a computer storage medium and a communication medium. The computer storage medium includes all of volatile and non-volatile media, and detachable and non-detachable media which are designed to store information including computer-readable commands, data structures, program modules, or other data. The communication medium includes computer-readable commands, a data structure, a program module, and other transmission mechanisms, and includes other information transmission media.

The foregoing exemplary embodiments and advantages are merely exemplary and are not to be construed as limiting. The present teaching can be readily applied to other types of apparatuses. The description of the exemplary embodiments is intended to be illustrative, and not to limit the scope of the claims, and many alternatives, modifications, and variations will be apparent to those skilled in the art. For example, each single component may be separated into multiple components which are then separately implemented. Also, separated components may be combined together or implemented as a single component.

## Claims

1. A method for providing a user interface (UI) for medical imaging of an object, the method comprising:
generating UIs respectively corresponding to processes for imaging the object;
determining a display order of the UIs comprising at least a first UI and a second UI of corresponding processes;
receiving a UI completion input of the medical professional regarding the first UI; and
displaying the second UI based on the determined display order in response to the receiving the UI completion input.

2. The method of claim 1, wherein the processes comprise at least two of a process of registering information of the object, a process of planning imaging, a process of controlling the imaging, and a process of displaying a result of the imaging.

3. The method of claim 1, further comprising:
displaying information about the processes in the determined display order on the UIs.

4. The method of claim 1, wherein the determining comprises receiving a signal for selecting one of imaging modes, from the medical professional,
the imaging modes are defined according to types of the processes for imaging the object, and
the selected imaging mode comprises at least one of a basic mode, an advanced mode, an analysis mode, and an automatic mode.

5. The method of claim 4, wherein the selected imaging mode comprises the automatic mode, in which pieces of information which have been input a number of times equal to or greater than a preset number are displayed on the UIs.

6. The method of claim 1, further comprising:
outputting an image of the object according to an input of the medical professional in the processes.

7. The method of claim 3, wherein the displaying the information comprises:
receiving a signal for selecting one of the processes; and
displaying the first UI corresponding to the selected process.

8. The method of claim 1, further comprising:
receiving an input completion signal for one of the processes, from the medical professional; and
displaying the first UI corresponding to a process that follows the one of the processes based on the determined display order of the UIs.

9. The method of claim 1, further comprising:
receiving a movement signal from the medical professional, while the second UI is displayed;
displaying a UI that follows the second UI based on the determined display order of the UIs, in response to the received movement signal being a forward movement signal; and
displaying the first UI, in response to the received movement signal being a backward movement signal.

10. A device comprising:
a memory configured to store a program comprising commands; and
a processor configured to execute the commands of the program to provide a user interface (UI) for medical imaging of an object, by:
generating UIs respectively corresponding to processes for imaging the object;
determining a display order of the UIs comprising at least a first UI and a second UI of corresponding processes;
receiving a UI completion input of a medical professional regarding a first UI; and
displaying a second UI based on the determined display order.

11. The device of claim 10, wherein information about the processes is displayed on the UIs in the determined display order.

12. The device of claim 10, wherein the determining comprises receiving a signal for selecting one of imaging modes,
the imaging modes are defined according to types of the processes for imaging the object, and
the selected imaging mode comprises at least one of a basic mode, an advanced mode, an analysis mode, and an automatic mode.

13. The device of claim 12, wherein the selected imaging mode comprises the automatic mode, in which pieces of information which have been input a number of times equal to or greater than a preset number are displayed on the UIs.

14. The device of claim 12, wherein the displaying comprises:
receiving an input completion signal for one of the processes, from the medical professional; and
displaying the first UI corresponding to a process that follows the one of the processes, based on the determined display order of the UIs.

15. The device of claim 10, wherein the displaying comprises:
receiving a movement signal from the medical professional;
displaying a UI that follows the second UI based on the determined display order of the UIs, in response to the received movement signal being a forward movement signal; and
displaying the first UI in response to the received movement signal being a backward movement signal.
